# EUROPEAN PATENT APPLICATION

(11) **EP 3 272 418 A1**
(43) Date of publication of application: **24.01.2018**
(21) Application number: 16180560.1
(22) Date of filing: 21.07.2016
(51) Int. Cl.: B01J 37/03, B01J 37/08, B01J 37/24, B01J 23/10, B01J 27/10, B01J 35/02, B01J 35/10, C07C 21/06

(54) **A CATALYST COMPOSITION FOR DIRECT SYNTHESIS OF VINYL CHLORIDE FROM ETHYLENE**

(71) Applicant: ETH Zurich, 8092 Zurich (CH)
(72) Inventor: PÉREZ-RAMIREZ, Javier, 8006 Zürich (CH); AMRUTE, Amol, 8051 Zürich (CH)

(57) **Abstract**

The present invention relates to a catalyst composition for one-step synthesis of vinyl chloride from ethylene comprising at least one rare earth metal M in the chlorinated forms MOCI or MCl₃. The invention relates also to a catalyst bed comprising said catalyst composition and a one-step process for the synthesis of vinyl chloride from ethylene.

## Description

The present invention relates to a catalyst composition for one-step synthesis of vinyl chloride from ethylene according to claim 1, a catalyst bed comprising said catalyst composition according to claim 11 and a process for a one-step synthesis of vinyl chloride from ethylene according to claim 14.

### Description

Polyvinyl chloride (PVC, 44 Mton per year) is an integral part of modern society owing to its widespread applications that improve our everyday life (Weissermel et al. Industrial Organic Chemistry, Wiley-VCH: Weinheim, 2008). Its monomer, vinyl chloride (VCM), was first produced on a commercial scale in the 1920s through a one-step route comprising acetylene hydrochlorination over mercuric chloride catalysts, which still is the main production method in countries, such as China, where coal is abundant. Around 1950, increasing demand and rising prices of acetylene prompted a shift in the feedstock to the more economical ethylene in the US and Europe. Since then, VCM is predominantly produced via a two-step process involving the oxychlorination of ethylene to ethylene dichloride (EDC) catalyzed by C_{U}Cl₂/γ-Al₂O₃ in fixed or fluidized-bed reactors and thermal cracking of EDC to VCM at temperatures above 500°C in a separate cracking reactor according to the following scheme:

(1) CH₂ = CH₂ + 2 HCl + 0.5 O₂ - ClCH₂CH₂Cl + H₂O

(2) ClCH₂CH₂Cl → CH₂ = CHCl + HCl

Although currently operated at an industrial scale, this process suffers from severe drawbacks, such as copper chloride volatilization, particle agglomeration and high energy costs due to the cracking step.

Significant effort has been dedicated to stabilize the CuCl₂-based active phase by doping with several additives or promoters. The promoters are those compounds which generally do not have reactivity in the oxychlorination process at the working conditions of the copper catalyst and are typically present in relatively low amounts compared to the active phase. They interact with the active phase and increase its melting point thereby increasing the stability or enhance the fluidization by reducing the degree of stickiness or change the surface acidity properties of the catalysts. Examples of such additives are alkali and alkaline earth metal compounds (US2010/0274061 A1, EP0582165 B2). Other additives from rare earth metal groups (most commonly lanthanum chloride) are also usually added to the catalyst composition (US4069170).

In contrast, new materials offering a long-term solution to the stability issues, are barely explored. The lack of robust materials also hampered the possible process intensification by integrating the two steps, thereby transforming ethylene directly to VCM over a catalyst that is stable at the high temperature needed for its formation.

In one approach rare earth metals were investigated as single catalyst for converting ethylene directly to vinyl chloride (US6909024B1). Here it was demonstrated that in particular lanthanum compound or lanthanum-containing compositions may be effective catalysts attaining high selectivity. Although lanthanum compounds were better than all other samples evaluated, they also were not very active as the reaction was carried out at very high space times (ca. 8-20 s, calculated by inventors as the volume of catalyst divided by the flow rate at standard conditions) in order to achieve a reasonable yield of VCM. This drawback of low yields hampered any feasibility for practical applications.

Recently, intense screening of oxide catalysts identified CeO₂ as possible alternative to improve the efficiency of the presently applied process (Scharfe et al., Angew. Chem. Int. Ed 2016, 55, 3068). CeO₂ was shown to be a stable catalyst for ethylene oxychlorination at a contact time (calculated by the same definition as above) of 0.14 s, yielding EDC (∼45% selectivity) and VCM (∼45% selectivity) in an equimolar ratio in a single pass at 400°C, i.e. 100-150°C lower than the temperature of the industrial thermal EDC cracking process. This bifunctional behaviour was attributed to the interaction of redox and acid properties where the intermediate EDC is formed on redox sites and subsequently dehydrochlorinated to VCM on acid sites. Although these were interesting findings compared to the traditional CuCl₂ system, CeO₂ alone has various disadvantages, such as the formation of over-chlorinated and over-oxidized by-products (ca. 10%). In addition, only 50% of the EDC produced can be transformed to VCM per pass.

Thus, there is still a need and desire to identify and develop catalytic systems with improved selectivity and yields for VCM synthesis that could reduce the currently high capital and operating costs of the PVC manufacturing process.

The object of the present invention is thus to provide a catalyst composition that allows the synthesis of vinyl chloride from ethylene in one step and at the same time shows increased selectivity and stability over the known systems.

This object is being solved by providing a catalyst composition with the features of claim 1 and a process for vinyl chloride synthesis from ethylene according to claim 14.

Accordingly, a catalyst composition for the one-step synthesis of vinyl chloride from ethylene comprising at least one rare earth metal M in the chlorinated forms MOCI or MCl₃.
wherein the chlorinated forms MOCI or MCl₃ are obtained by contacting at least one corresponding rare earth metal oxide with a HCl stream, preferably with a contact time between 0.05 and 1 s, in particular preferably between 0.1 and 0.5 s,
wherein the at least one corresponding rare earth metal oxide underwent a thermal pretreatment at temperatures between 300 and 1300°C, preferably between 500 and 900°C before the HCl contact.

The present catalyst composition enables the direct synthesis of vinyl chloride from ethylene in the presence of HCl and O₂ (or air). The present catalyst composition transforms ethylene in the presence of HCl and O₂ (or air) via ethylene dichloride to vinyl chloride. Thus, the oxychlorination of ethylene and dehydrochlorination of ethylene dichloride take place in a single step at moderate temperatures below 500°C. The present catalyst composition avoids thus the conventional two-step synthesis of vinyl chloride involving oxychlorination followed by highly energy-intensive cracking (typically above 500°C) in two separate reactors.

It is to be understood that the chlorinated forms are the catalytically active forms of the rare earth metal M, in particular in case when using a single rare earth metal. Thus, the chlorinated forms of the rare earth metal are obtained by conversion of the respective rare earth metal oxide in the presence of HCl. Depending on the type of rare earth metal this provides the oxychloride compound MOCI or the fully chlorinated compound MCl₃.

In one embodiment the rare earth metal oxide used for chlorination may be commercially available.

However, in a more preferred embodiment the rare earth metal oxide is obtained by providing a solution of at least one rare earth metal salt, in particular nitrate, carbonate or halides, for example in water, adding at least one N-containing base, in particular NH₄OH (but no KOH or NaOH) in the presence of at least one peroxide, in particular H₂O₂, for precipitating the corresponding rare earth metal hydroxide, followed by collecting and thermally treating the precipitate to obtain a oxide form.

Thus, the rare earth metal oxide is provided in a multi-step process starting with the corresponding salt, forming the respective hydroxide in the presence of a peroxide compound followed by thermal treatment. As later described in more detail, it is also possible to use a mixture of multiple rare earth metal salts, such two different rare earth metal salts, as starting material that may be precipitated together.

In one further embodiment the present catalyst composition is substantially free or devoid of any further metal, in particular free of any copper (in form of copper oxide or copper salt), alkali metal salt and/or alkaline earth metal salt (e.g. magnesium, calcium). It is to be understood that in the context of the present invention substantially free or devoid includes an amount or less that would not have an appreciable impact on the catalyst response of the material.

The rare earth metals M that may be in general employed are preferably elements of the lanthanide series listed as elements 57 through 71 of the Periodic Table. Examples of rare earth metals include lanthanum (La), cerium (Ce), praseodymium (Pr), neodymium (Nd), samarium (Sm), europium (Eu), gadolinium (Gd), terbium (Tb), dysprosium (Dy), holmium (Ho), erbium (Er) and ytterbium (Yb). The preferred rare earth metals are lanthanum, neodymium, gadolinium, praseodymium, dysprosium and europium, whereby europium is the most preferred rare earth metal.

In a preferred embodiment the following rare earth metals are present in the catalyst in their oxychloride form: PrOCl, DyOCl, GdOCl and EuOCl, whereby EuOCl is the most preferred catalyst.

In a yet other embodiment the following rare earth metals are present in the catalyst in their chloride form: NdCl₃ and LaCl₃.

As previously described the chlorinated forms of the rare earth metal (MOCI, MCl₃) are obtained from their respective oxides in the presence of HCl and oxygen. For example La₂O₃ is converted to LaCl₃, Pr₄O₇ to PrOCl, Nd₂O₃ to NdCl₃, Eu₂O₃ to EuOCl, Gd₂O₃ to GdOCl, and Dy₂O₃ to DyOCl.

As mentioned above the rare earth metal oxide is calcined or thermally pretreated (or activated) before chlorination in the HCl stream. Accordingly, the rare earth metal oxides are calcined or thermally pretreated before HCl contact at temperatures between 300 and 1300°C, preferably between 500 and 900°C. For example, the commercially available rare earth metal oxide may be thermally pretreated at 500°C, while the oxides obtained via precipitation may be calcined or pretreated at 500°C, 700°C or 900°C.

In most preferred embodiment the at least one rare earth metal oxide is an oxide of europium undergoing a thermal treatment at 500°C, 700°C and 900°C. These calcined forms are subsequently converted to their oxychloride forms EuOCl (500, 700, 900°C).

The particle size of the chlorinated rare earth metal compounds (obtained from electron microscopy and X-ray diffraction analyses) may be (in diameter) between 10 and 1000 nm, preferably between 50 and 500 nm, more preferably between 100 and 300 nm. The particle size may vary depending on the rare earth metal. For example EuOCl may be present in form of nanoparticles of a size about 40 nm, whereas LaCl₃ may form agglomerates with a size between 100-200 nm.

The specific surface area of the at least partially chlorinated rare earth metal compounds (obtained using the well-known BET (Brunauer-Emmett-Teller) method to the N₂ isotherm at -196°C) depends strongly on the surface area of the starting material (here rare earth metal oxides). In general the total surface area (m²/g) of the at least partially chlorinated rare earth metal compounds may be in a range between 5 and 50 m²/g, preferably between 10 and 30 m²/g, more preferably between 15 and 25 m²/g. The most preferred EuOCl may have a surface area between 8 and 26 m²/g depending on the previously applied calcination temperature.

In one variant of the present catalyst composition a mixed composition of two, three, four or more rare earth metal oxides is contacted with the HCl stream. Thus, the chlorinated rare earth metal compounds, in particular MOCI or MCl₃, may be used as single compound in the present catalyst composition, i.e. they may contain only one rare earth metal compound, or in form of a mixture of multiple rare earth metal compounds.

In a preferred embodiment of the present catalyst composition a mixed composition of europium oxide and cerium oxide Eu*ₓ*Ce_{1-*x*}O₂₋₀._{5*x*} with *x* ≤ 0.5, in particular Eu_{0.4}Ce₀.₆O₁.₈ is contacted with the HCl stream. Said mixture is obtained by co-precipitating the respective rare earth metal salts using a N-containing base in the presence of a peroxide. The mixed oxides system is subsequently chlorinated in the HCl stream. Thereby a catalyst system is obtained that preserves the bulk as oxide likely due to strong interactions between europium and cerium, while on the surface both oxide and oxychloride phases co-exist thus leading to efficient oxychlorination and dehydrochlorination functions.

In another embodiment of the present catalyst composition the at least one at least partially chlorinated rare earth metal compound is dispersed on a support or solid carrier. Such carriers and supports are generally known. It is in particular conceivable to use a support that may act as an additive for promoting stability as well as selectivity to desired product. The supported catalyst may be obtained by methods known by those skilled in the art such as impregnation and successfully used in ethylene oxychlorination.

The present catalyst composition may be used in at least one catalyst bed in a suitable reactor.

In one embodiment the catalyst bed may contain the present catalyst compositions (i.e. singular compound, mixed compounds, compound on a support) in one phase.

In another embodiment it is also possible that the present catalyst bed is provided in form of a dual-catalyst bed comprising at least one first chlorinated rare earth metal compound and at least one second other rare earth metal compound. In this case the second other rare earth metal compound may be also a chlorinated rare metal earth compound or may be a rare earth metal oxide.

In said dual-catalyst bed the at least one first chlorinated rare earth metal compound and the at least one second other rare earth metal compound are arranged as separate phases. Both compounds are not dispersed or mixed with each other, but form (spatially) separated phases on top of each other. This means that for example the first partially chlorinated rare earth metal compound may be arranged downstream (below) the second other rare earth metal compound. The mass ratio of both phases may be in a range between 0.5 : 1.5 and 1.5 : 0.5, preferably 1 : 1.

In a most preferred embodiment the catalyst bed comprises a MOCI phase, in particular a EuOCl phase, and a rare earth metal oxide phase, in particular a CeO₂ phase. Thus, the first chlorinated rare earth metal compound may be EuOCl and the other second rare earth metal compound may be CeO₂. In this case the EuOCl phase is provided downstream of the CeO₂ phase in the direction of the gas flow, i.e. the CeO₂ is arranged on top of EuOCl in flow direction of the gas feed (for example comprising ethylene, HCl, oxygen, and a carrier (inert) gas). This means that the reactive gas stream enters at first the CeO₂ phase for the oxychlorination of ethylene to ethylene dichloride and subsequently the EuOCl phase for the dehydrochlorination of ethylene dichloride to vinyl chloride.

The present catalyst composition is used in a process for synthesizing vinyl chloride from ethylene in one step. The one-step process comprises the step of contacting a gas feed containing a mixture of ethylene, oxygen or oxygen containing gas, and hydrogen chloride with the present catalyst composition, in particular with the present catalyst bed.

In an embodiment of the present process the gas feed mixture contains 1-5 vol.%, preferably 3 vol.% ethylene, 1-6 vol.%, preferably 3-5 vol.%, more preferably 4-4.8 vol.% HCl and 1-6 vol.%, preferably 2-5 vol.%, more preferably 3-4 vol.% O₂ balanced in an inert gas, such as helium, argon, nitrogen or any other suitable gas.

The reaction temperature of the present process, in particular the temperature in the catalyst bed is between 300 and 500°C, preferably between 350 and 500°C.

The further process parameters are as follows. The process pressure may be between 0.1 MPa and 10 MPa, preferably between 0.5 and 5 MPa. The contact time of the gas feed with the catalyst composition is between 0.05 and 1 s, preferably between 0.1 and 0.6 s (for example between 0.07 and 0.56 s).

The invention is now explained in more detail by means of examples with reference to the figures. It shows:

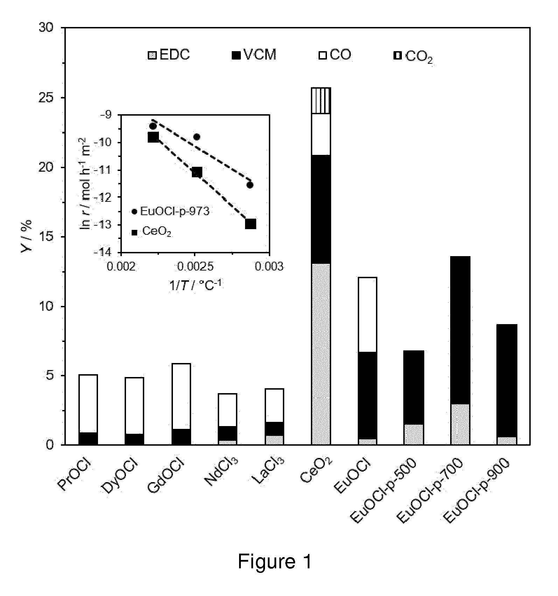

- Figure 1: Product yields (*Y*) in ethylene oxychlorination over lanthanide catalysts. The inset depicts the Arrhenius plot of VCM formation over EuOCI-p-700 and CeO₂. Conditions: Temperature, *T* = 450°C, feed mixture = 3 vol.% C₂H₄, 4.8 vol.% HCl, 3 vol.% O₂, and 3 vol.% Ar in He, contact time, r= 0.14 s.

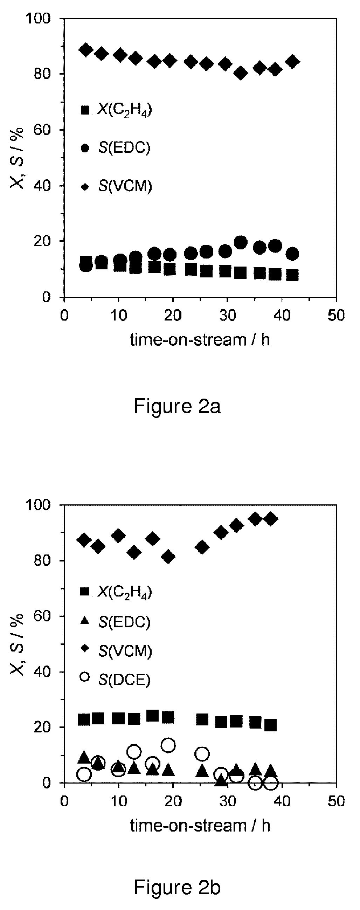

- Figure 2a,b: C₂H₄ conversion and product selectivities versus time-on-stream in ethylene oxychlorination over a) EuOCI-p-700 and b) Eu_{0.4}Ce_{0.6}O_{1.8}. Conditions: T= 450°C (400°C for Eu_{0.4}Ce_{0.6}O_{1.8}), feed mixture = 3 vol.% C₂H₄, 4.8 vol.% HCl, 3 vol.% O₂, and 3 vol.% Ar in He, r= 0.14 s.

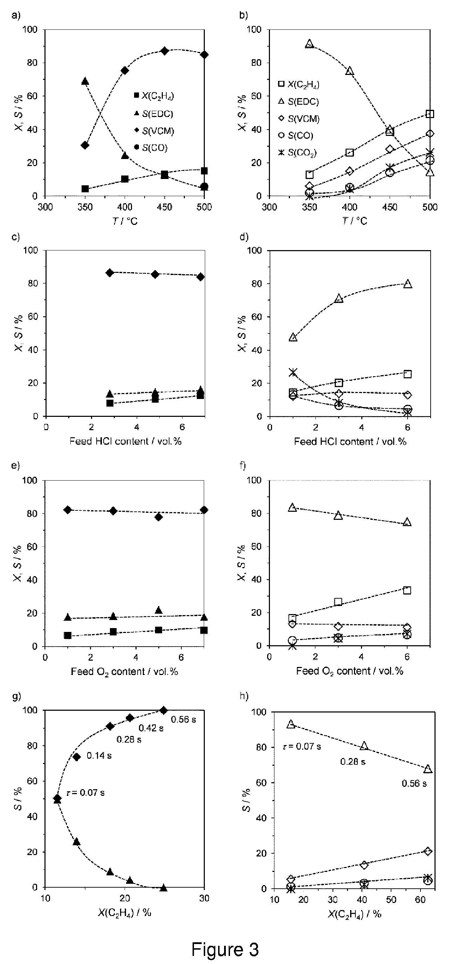

- Figure 3: C₂H₄ conversion and product selectivity over EuOCI-p-700 (a,c,e,g) and CeO₂-900 (b,d,f,h) as a function of a,b) T at fixed HCl (4.8 vol.%) and O₂ (3 vol.%) concentrations, c,d) feed HCl content at fixed O₂ content (3 vol.%) at 450°C (EuOCl) and 400°C (CeO₂), and e,f) feed O₂ content at fixed HCl content (4.8 vol.%) at 450°C (EuOCl) and 400°C (CeO₂). g,h) Selectivity to products versus conversion at different contact times at 450°C (EuOCl) and 400°C (CeO₂) at fixed HCl (4.8 vol.%) and O₂ (3 vol.%) concentrations, symbol as in a-c). Conditions: r= 0.14 s.

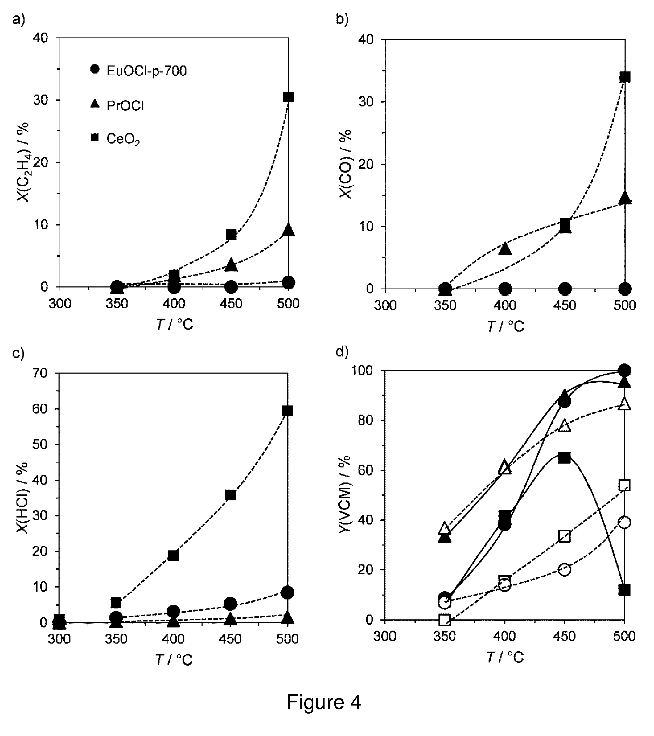

- Figure 4: Performance descriptors for selective VCM production over used catalysts. a) C₂H₄ oxidation to CO₂ (3 vol.% C₂H₄, 3 vol.% O₂), b) CO oxidation to CO₂ (2.5 vol.% CO, 2.5 vol.% O₂), c) HCl oxidation to Cl₂ (3 vol.% HCl, 3 vol.% O₂) d) EDC dehydrochlorination to VCM (Open symbols: 1.5 vol.% EDC and solid symbols: 1.5 vol.% EDC, 4.8 vol.% HCl, 3 vol.% O₂). Condition: r= 0.14 s.

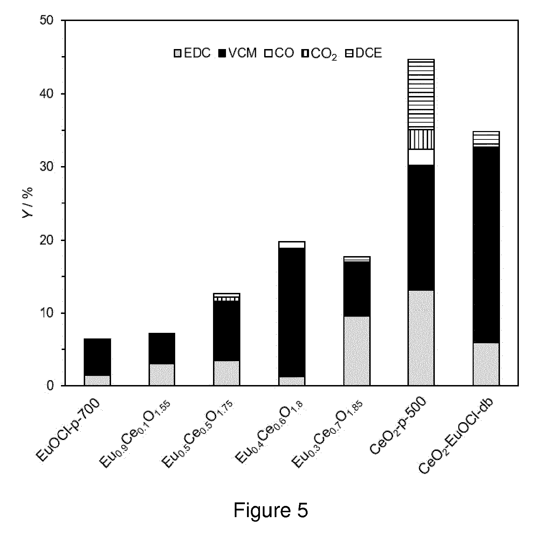

- Figure 5: Product yields (*Y*) in ethylene oxychlorination over Eu*ₓ*Ce_{1-*x*}O₂₋₀._{5*x*} catalysts and double bed catalyst. Conditions: T= 400°C, feed mixture = 3 vol.% C₂H₄, 4.8 vol.% HCl, 3 vol.% O₂,3 vol.% Ar in He, r= 0.14 s.

### Example 1

Commercial La₂O₃ (Alfa Aesar, 99.99%), CeO₂ (Sigma-Aldrich, 99.9%), Pr₂O₃ (Alfa Aesar, 99.9%), Nd₂O₃ (Sigma-Aldrich, 99.9%), Eu₂O₃ (Sigma-Aldrich, 99.5%), Gd₂O₃ (Alfa Aesar, 99.99%), and Dy₂O₃ (ABCR, 99.99%) were treated at 500°C in static air using a heating rate of 5°C min⁻¹ and an isothermal step of 5 h prior to their use in catalysis. Analysis by X-ray diffraction confirmed the corresponding oxide phases, except for the commercial praseodymium oxide which actually consisted of Pr₄O₇ and thus it is denoted as such in the description of the present invention. The total surface area determined by N₂ sorption analysis, presented in Table 1, ranged from 5-45 m²/g. The solids were mechanically pressed into pellets which were subsequently crushed and sieved to achieve particles in the range of 0.4-0.6 mm.

The gas-phase oxychlorination of ethylene was carried out at ambient pressure in a continuous-flow fixed-bed reactor. The set-up consists of (*i*) mass flow controllers to feed C₂H₄ (PanGas, 20.15% in He), HCl (Air Liquide, purity 2.8, anhydrous), O₂ (Messer, 10.06% in He), He (PanGas, purity 5.0) as a carrier gas, and Ar (PanGas, purity 5.0) as an internal standard, (*ii*) a syringe pump (Nexus 6000, Chemyx) to feed EDC (Fluka, 99.5%), *(iii)* a vaporizer operated at 130°C accommodating a quartz T-connector filled with glass beads to vaporize EDC, (*iv*) an electrically heated oven hosting a quartz micro-reactor equipped with a K-type thermocouple whose tip reaches the center of the catalyst bed, (v) downstream heat tracing to avoid any condensation of the reactants and products, and *(vi)* a gas chromatograph coupled to mass spectrometer (GC-MS) for on-line analysis.

The sieved catalyst particles were loaded into the micro-reactor (10 mm inner diameter) and pre-treated in He at 200°C for 30 min. Thereafter, a reaction feed composed of a 3 vol.% C₂H₄, 1-6 vol.% HCl, and 1-6 vol.% O₂, balanced in He was fed to the reactor at a contact time of 0.07-0.56 s and a bed temperature (*T*) of 350-500°C. The XRD analysis of catalysts after the oxychlorination tests indicated that the starting oxides are transformed into oxychloride (MOCI) or chloride (MCl₃) forms, except in the case of CeO₂ which preserved its oxide phase. In particular, Pr₄O₇, Dy₂O₃, Gd₂O₃, and Eu₂O₃ were transformed to PrOCl, DyOCl, GdOCl, and EuOCl, respectively, while La₂O₃ and Nd₂O₃ were transformed to LaCl₃ and NdCl₃. This means actual catalysts for oxychlorination, except for the case of CeO₂, were oxychlorides and chlorides and therefore they are called in the description and in illustrations by these phases. The yield of product i, *Y*(*i*), calculated by the product of ethylene conversion and selectivity to product i, for different compositions is summarized in Figure 1.

The overall VCM yield differs between the compound tested. It is to be noted that the chlorinated lanthanides shown in the diagram of Figure 1 show catalytic activity providing VCM with no or small percentage of EDC whereas CeO₂ results in a rather high yield of EDC. The best performing catalyst with a lower perecentage yield of EDC and high percentage yield of VCM are EuOCl (VCM 6.21; EDC 0.5), EuOCl-p-500 (VCM 5.2; EDC 1.5), EuOCI-p-700 (VCM 10.5; EDC 3.0), EuOCl-p-900 (VCM 5.2; EDC 0.6),

It should be noted that each catalytic data point reported in Figure 1 and in the further examples is an average of at least three measurements. The carbon mass balance error in all catalytic tests was below 4%.

### Example 2

High surface area europium oxide (Eu₂O₃-p-*T*_{cal}, where *T*_{cal} denotes the calcination temperature in °C) was synthesized by precipitation (p) route in the presence of H₂O₂ following a protocol reported elsewhere (Scharfe et al., Angew. Chem. Int. Ed. 2016, 55, 3068).

The presence of H₂O₂ during synthesis improves the morphological, redox, and thermal stability properties of the yielding solid product.

H₂O₂, actually, decomposes the precursor to form metal hydroperoxide complexes. This disrupts the formation of a compact M-O-M network during the crystallization process, favoring the formation of highly-dispersed nanocrystallites of metal oxide (Pappacena et al., Int. J. Hydrogen Energy 2012, 37, 1698; Stud. Surf. Sci. Catal. 2010, 175, 835).

Eu₂(NO₃)₃-6H₂O (ABCR, 99.9%) was dissolved in deionized water under stirring and H₂O₂ (Acros Organics, 35%) was added to the solution to obtain a molar H₂O₂:*M* ratio of 3 (M= Eu). The precipitation was achieved by the dropwise addition of aqueous NH₄OH (Sigma-Aldrich, 30%) until a pH of 10.5 was reached. The slurry was stirred for 4 h and washed with deionized water. Upon filtration, the precipitate was dried at 120 for 12 h and calcined at 500-900°C using 5°C min⁻¹ ramp in flowing air for 5 h. The X-ray diffraction analysis for obtained solid confirmed the Eu₂O₃ phase in all the three samples. The total surface area, given in Table 1, was 37, 31, and 14 m²/g for samples calcined at 500°C, 700°C, and 900°C, respectively. The powders were mechanically pressed into pellets which were subsequently crushed and sieved to achieve particles in the range of 0.4-0.6 mm.

The sieved particles were evaluated in the oxychlorination of ethylene using the reactor set-up and protocol described in Example 1. In this case also the starting oxide is transformed into oxychloride EuOCl phase (and thus are denoted as EuOCl-p-*T*_{cal}). The yield of product *i*, *Y*(*i*), calculated by the product of ethylene conversion and selectivity to product *i*, for these europium oxychloride samples is summarized in Figure 1 and the rate of VCM production, *r*, expressed as mole of VCM per unit of time and catalyst surface area, for EuOCI-p-700 and CeO₂ is given in inset of Figure 1. The time-on-stream performance of EuOCI-p-700 is shown in Figure 2a.

### Example 3

The performance of EuOCI-p-700, which stand out as the best catalyst composition to attain highest VCM yield and no CO*ₓ* formation, is contrasted to previously reported CeO₂ catalyst (Scharfe et al., Angew. Chem. Int. Ed. 2016, 55, 3068). This comparison will emphasize the superiority of EuOCl composition. The Eu₂O₃-p-700 was obtained by procedure reported in Example 2 and converted to EuOCI-p-700 in the reaction. Commercial CeO₂ (Sigma-Aldrich, 99.9%) was treated at 900°C in static air using a heating rate of 5°C min⁻¹ and an isothermal step of 5 h. This sample is denoted as CeO₂-900, like in Scharfe et al., Angew. Chem. Int. Ed. 2016, 55, 3068. The powders were mechanically pressed into pellets which were subsequently crushed and sieved to achieve particles in the range of 0.4-0.6 mm.

The sieved catalysts were evaluated in the oxychlorination of ethylene using the reactor set-up and protocol described in Example 1. The comparison is made under different conditions, thus by varying reaction temperature (diagrams a,b), feed HCl contents (diagrams c,d), feed O₂ contents (diagrams e,f), and contact times (diagrams g,h). The results are displayed in Figure 3. The diagrams a, c, e and g depict the results obtained for EuOCI-p-700 while diagrams b, d, f and h depict the results for CeO₂. As clearly deducible EuOCI-p-700 has a higher selectivty for VCM conpared to CeO₂.

### Example 4

The superior performance of EuOCI-p-700 composition is explained through conducting oxidation tests involving two reactants, i.e. C₂H₄ and O₂ or CO and O₂ or HCl and O₂, and by EDC dehydrochlorination activity. In all cases the performance of EuOCI-p-700 is contrasted with PrOCl and CeO₂. Eu₂O₃-p-700 was prepared by protocol reported in Example 2, while Pr₄O₇ and CeO₂ were obtained by procedure reported in Example 1.

The powders were mechanically pressed into pellets which were subsequently crushed and sieved to achieve particles in the range of 0.4-0.6 mm. The sieved catalysts were exposed to ethylene oxychlorination conditions using the reactor set-up and protocol described in Example 1 and allowed to equilibrate for 3 hours. This procedure yield EuOCI-p-700, PrOCl, and surface chlorinated CeO₂, which were tested in explanatory tests using set-up described in Example 1.

The oxidation tests were conducted at a contact time of 0.14 s and T in the range of 250-500°C by feeding 3 vol.% C₂H₄, 3 vol.% O₂, and 3 vol.% Ar balanced in He for ethylene oxidation to CO₂ or by feeding 2.5 vol.% CO, 2.5 vol.% O₂, and 3 vol.% Ar balanced in He for CO oxidation to CO₂ or by feeding 3 vol.% HCl, 3 vol.% O₂, and 3 vol.% Ar balanced in He for HCl oxidation to Cl₂. In the EDC dehydrochlorination tests, 1.5 vol.% EDC was fed using He as carrier gas with or without addition of HCl and O₂ at a contact time of 0.14 s and T in the range of 250-500°C. The results of ethylene oxidation are shown in Figure 4a, of CO oxidation in Figure 4b, of HCl oxidation in Figure 4c, and of EDC dehydrochlorination in Figure 4d.

### Example 5

The superior activity (ethylene conversion) of CeO₂ to EuOCl was combined with outstanding VCM productivity of EuOCl to yield a catalyst that will lead to improved yield of VCM. Thus, mixed europium-cerium oxides (Eu*ₓ*Ce_{1-*x*}O₂₋₀._{5*x*}, where *x* represents the mole fraction of Eu) were synthesized by coprecipitation method following a protocol reported in Example 2, where cerium nitrate was added to the aqueous solution of europium nitrate prior to the addition of H₂O₂. The further execution of the precipitation process was kept identical as described above, until the calcination at 500°C in flowing air for 5 h. The XRD analysis of sample showed the presence of oxide phase. Since the reflections of Eu₂O₃ and CeO₂ have matching position, thus they could not be distinguished by XRD in these mixed systems. Eu:Ce ratio present in each composition and total surface area were determined by X-ray fluorescence and N₂ sorption analysis, respectively. These results are presented in Table 2. The powder samples were sieved to achieve particles in the range of 0.4-0.6 mm.

The sieved catalysts were evaluated in the oxychlorination of ethylene using the reactor set-up and protocol described in Example 1. The yield of product *i*, *Y*(*i*), calculated by the product of ethylene conversion and selectivity to product *i*, for different compositions is summarized in Figure 5. The Eu_{0.4}Ce_{0.6}O_{1.8} found as the best composition reaching highest VCM yield. The time-on-stream performance of Eu_{0.4}Ce_{0.6}O_{1.8} is shown in Figure 2b. In case of Eu_{0.4}Ce_{0.6}O_{1.8} the high selectivity of EuOCl for VCM could be maintained while the overall VCM yield was increased in comparison to EuOCl (see diagrams of Figures 2a and 2b).

The compositions of mixed systems after the oxychlorination tests were analyzed by XRD. It was found that all compositions where *x* ≤ 0.5 in the general mixed composition formula Eu*ₓ*Ce_{1-*x*}O_{2-0.5*x*} have the oxide phase after the oxychlorination test. The analysis of surface by X-ray photoelectron spectroscopy of a selected composition with *x* = 0.4 showed the presence of chorine on the surface. Thus, in case of mixed systems where *x* ≤ 0.5, only the surface is chlorinated and might have an oxychloride phase, while bulk remained as oxide.

### Example 6

In another approach to further improve the VCM yield, a double catalyst bed concept was applied. Herein, to couple the high productivity of CeO₂ with the high EDC dehydrochlorination ability of EuOCl, the first catalyst bed comprised of CeO₂-p-500 (obtained by precipitation of cerium nitrate with procedure described in Example 2, followed by calcination at 500°C) is separated from a second EuOCI-p-700 (obtained according to Example 2) bed.

The above combination was evaluated in the oxychlorination of ethylene using the reactor set-up and protocol described in Example 1. The yield of product *i*, *Y*(*i*), calculated by the product of ethylene conversion and selectivity to product *i*, is presented in Figure 5. Indeed, this system resulted in the highest VCM and VCM+EDC yields. The double bed system of CeO₂-EuOCl provides a VCM yield of 26.6 and a EDC yield of 6.

**Table 1: Total surface area of samples prior (fresh) and after (used) oxychlorination reaction.**

| **Fresh sample^{a}** | ***S*_{BET}^{b} (m2 g⁻¹)** | **Used sample** | ***S*_{BET}^{b} (m² g⁻¹)** |
|---|---|---|---|
| La₂O₃ | 6 | LaCl₃ | 8 |
| CeO₂ | 45 | CeO₂ | 28 |
| Pr₄O₇ | 5 | PrOCl | 6 |
| Nd₂O₃ | 32 | NdCl₃ | 21 |
| Eu₂O₃ | 11 | EuOCl | 8 |
| Eu₂O₃-p-500 | 37 | EuOCI-p-500 | 26 |
| Eu₂O3-p-700 | 31 | EuOCI-p-700 | 23 |
| Eu₂O₃-p-900 | 14 | EuOCI-p-900 | 12 |
| Gd₂O₃ | 5 | GdOCl | 16 |
| Dy₂O₃ | 29 | DyOCl | 25 |

| | | | |
|---|---|---|---|
| ^{a} All samples were commercial, except Eu₂O₃-p-*x*, which was obtained by precipitation and calcination at *x* °C. ^{b} BET method. | | | |

**Table 2: Characterization data of the mixed europium-cerium oxides.**

| **Sample** | **Eu:Ce^{a} (mol mol⁻¹)** | ***S*_{*B*ET}^{b} (m² g⁻¹)** |
|---|---|---|
| Eu₂O₃-p-700 | 1:0 | 31 |
| Eu_{0.9}Ce_{0.1}O_{1.55} | 0.9:0.1 | 16 |
| Eu_{0.5}Ce_{0.5}O_{1.75} | 0.51:0.49 | 13 |
| Eu_{0.1}Ce_{0.6}O_{1.8} | 0.39:0.61 | 27 |
| Eu_{0.3}Ce_{0.7}O_{1.85} | 0.3:0.7 | 25 |
| CeO₂-p-500 | 0:1 | 44 |

| | | |
|---|---|---|
| ^{a}XRF. ^{b}BET method. | | |

## Claims

1. A catalyst composition for one-step synthesis of vinyl chloride from ethylene comprising at least one rare earth metal M in the chlorinated forms MOCI or MCl₃
**characterized in that**
the chlorinated forms MOCI or MCl₃ are obtained by contacting at least one corresponding rare earth metal oxide with a HCl stream, preferably with a contact time between 0.05 and 1 s, in particular preferably between 0.1 and 0.6 s,
wherein the at least one corresponding rare earth metal oxide underwent a thermal pretreatment at temperatures between 300 and 1300°C, preferably between 500 and 900°C before the HCl contact.

2. Catalyst composition according to claim 1, **characterized in that** the rare earth metal oxide is obtained by providing a solution of at least one rare earth metal salt, precipitating the corresponding rare earth metal hydroxide by adding at least one N-containing base in the presence of at least one peroxide, collecting and thermally treating the precipitate.

3. Catalyst composition according to claim 1 or 2, **characterized in that** the catalyst composition is substantially free of any further metal, alkali metal salt and/or alkaline earth metal salt.

4. Catalyst composition according to one of the preceding claims, **characterized in that** the rare earth metals are selected from a group comprising lanthanum, neodymium, gadolinium, praseodymium, dysprosium and europium.

5. Catalyst composition according to one of the preceding claims, **characterized in that** the oxychlorinated form MOCI comprises PrOCl, DyOCl, GdOCl and EuOCl.

6. Catalyst composition according to one of the preceding claims, **characterized in that** the chlorinated form MCl₃ comprises NdCl₃ and LaCl₃.

7. Catalyst composition according to one of the preceding claims, **characterized in that** the at least one rare earth metal oxide is an oxide of europium undergoing a thermal treatment at 500°C, 700°C and 900°C.

8. Catalyst composition according to one of the preceding claims, **characterized in that** a mixed composition of two, three, four or more rare earth metal oxides is contacted with the HCl stream.

9. Catalyst composition according to claim 8, **characterized in that** a mixed composition of europium oxide and cerium oxide Eu*ₓ*Ce_{1-*x*}O_{2-0.5*x*} with *x* ≤ 0.5, in particular Eu_{0.4}Ce_{0.6}O_{1.8} is contacted with the HCl stream.

10. Catalyst composition according to one of the preceding claims, **characterized in that** the at least one chlorinated rare earth metal compound is dispersed on a support or solid carrier.

11. Catalyst bed for a reactor for the one-step synthesis of vinyl chloride from ethylene comprising a catalyst composition according to one of the preceding claims.

12. Catalyst bed according to claim 11, **characterized by** a double catalyst bed comprising at least one first chlorinated rare earth metal compound and at least one second other rare earth metal compound.

13. Catalyst bed according to claim 11 or 12, **characterized by** a dual catalyst bed comprising a MOCI phase, in particular a EuOCl phase, and a rare earth metal oxide phase, in particular a CeO₂ phase.

14. Process for synthesizing vinyl chloride from ethylene in one-step comprising the step of contacting a gas feed containing a mixture of ethylene, oxygen or oxygen containing gas and hydrogen chloride with a catalyst composition according to one of the claims 1-10, in particular with a catalyst bed according to one of the claims 11-13.

15. Process according to claim 14, **characterized in that** the gas feed mixture contains 1-5 vol.%, preferably 3 vol.% ethylene, 1-6 vol.% HCl and 1-6 vol.% O₂ balanced in an inert gas, in particular helium, argon, nitrogen or any other suitable gas.

16. Process according to one of the claims 14-15, **characterized in that** the reaction temperature is between 300 and 500°C, preferably between 350 and 500°C.

17. Process according to one of the claims 14-16, **characterized in that** a contact time of the gas feed with the catalyst composition is between 0.05 and 1 s, preferably between 0.1 and 0.6 s.
